# EUROPEAN PATENT SPECIFICATION

(11) **EP 2 440 574 B1**
(45) Date of publication and mention of the grant of the patent: **26.05.2021**
(21) Application number: 09787605.6
(22) Date of filing: 23.07.2009
(51) Int. Cl.: C07K 14/445, C07K 1/14, C07K 1/22, A61K 39/00, A61K 39/002, A61K 39/015

(54) **STABLE IMMUNOGENIC PROTEIN HAVING MULTIPLE CYSTEINES MOLECULES PROCESS THEREFOR AND COMPOSITION THEREOF**
STABILES IMMUNOGENES PROTEIN MIT MEHREREN CYSTEINMOLEKÜLEN, VERFAHREN DAFÜR UND ZUSAMMENSETZUNG DAVON
PROTÉINE IMMUNOGÈNE STABLE CONTENANT PLUSIEURS MOLÉCULES DE CYSTÉINE, SON PROCÉDÉ ET SA COMPOSITION

(30) Priority: 09.06.2009 IN 1356CH2009
(43) Date of publication of application: 18.04.2012
(60) Divisional application: 20158316.8
(73) Proprietor: Bharat Biotech International Limited, Hyderabad 500 078 (IN)
(72) Inventor: ELLA, Krishna Murthy, Shameerpet 500 078 Hyderabad (IN); RAVI , Ganapathy, Hyderabad 500 078 (IN)
(74) Representative: dompatent von Kreisler Selting Werner - Partnerschaft von Patent- und Rechtsanwälten mbB
(86) International application number: PCT/IN2009/000417
(87) International publication number: WO 2010/143194

(56) References cited:
- SYED SHAMS YAZDANI ET AL: "A high cell density fermentation strategy to produce recombinant malarial antigen in E. coli" BIOTECHNOLOGY LETTERS, KLUWER ACADEMIC PUBLISHERS, DO, vol. 26, no. 24, 1 December 2004 (2004-12-01), pages 1891-1895, XP019230824 ISSN: 1573-6776
- PALMER I ET AL: "Preparation and Extraction of Insoluble (Inclusion-Body) Proteins from Escherichia coli" CURRENT PROTOCOLS IN PROTEIN SCIENCE, JOHN WILEY & SONS, INC, US, vol. Chapter 6, 1 November 2004 (2004-11-01), pages 6.3.1-6.3.18, XP009131310 ISSN: 1934-3655
- WILHARM ELKE ET AL: "Generation of catalytically active granzyme K from Escherichia coli inclusion bodies and identification of efficient granzyme K inhibitors in human plasma" JOURNAL OF BIOLOGICAL CHEMISTRY, AMERICAN SOCIETY FOR BIOCHEMISTRY AND MOLECULAR BIOLOGY, INC, US, vol. 274, no. 38, 17 September 1999 (1999-09-17), pages 27331-27337, XP002158680 ISSN: 0021-9258
- RUDOLPH R ET AL: "IN VITRO FOLDING OF INCLUSION BODY PROTEINS" FASEB JOURNAL, FED. OF AMERICAN SOC. FOR EXPERIMENTAL BIOLOGY, BETHESDA, MD, US, vol. 10, no. 1, 1 January 1996 (1996-01-01), pages 49-56, XP000605154 ISSN: 0892-6638
- YAZDANI S S ET AL: "Evaluation of immune responses elicited in mice against a recombinant malaria vaccine based on Plasmodium vivax Duffy binding protein" VACCINE, BUTTERWORTH SCIENTIFIC. GUILDFORD, GB, vol. 22, no. 27-28, 9 September 2004 (2004-09-09), pages 3727-3737, XP004526954 ISSN: 0264-410X
- CLELAND J L ET AL: "A SPECIFIC MOLAR RATIO OF STABILIZER TO PROTEIN IS REQUIRED FOR STORAGE STABILITY OF A LYOPHILIZED MONOCLONAL ANTIBODY" JOURNAL OF PHARMACEUTICAL SCIENCES, AMERICAN PHARMACEUTICAL ASSOCIATION, WASHINGTON, US, vol. 90, no. 3, 1 March 2001 (2001-03-01), pages 310-321, XP001179875 ISSN: 0022-3549

## Description

### FIELD OF INVENTION

The invention relates to life sciences particularly biomedical research more particularly to Translational Research in order to develop alternate therapeutic agents as preventive medicine. More specifically, the invention provides a stable immunogenic protein, and a composition containing said protein useful as vaccine having increased purity, stability, immunogenicity, without impairing antigen stability, integrity, and functionality. The vaccine thus obtained is preferably effective against malarial infections using technically & commercially viable and/or industrially feasible process. Particularly, the invention provides an industrially scalable, high yielding, cost effective process for expression, purification and refolding of protein having multiple cysteine molecules enhanced shelf life and immunogenicity. Further, the invention in particular relates to expression, purification and refolding of rPvRII having 12 cysteines useful for prophylaxis of malarial infections in mammals more specifically malarial infections caused by *Plasmodium sp.* The invention provides unique composition having high antibody titre and enhanced shelf life up to three years.

### BACKGROUND OF THE INVENTION:

Recombinant proteins, when expressed in high levels in organisms like *E. coli* aggregate together to form insoluble matter, called popularly as inclusion bodies. These inclusion bodies have to be brought into solution by using solubilizing agents. After this they have to be purified partially or fully before subjecting the protein to refolding / renaturation thereby enabling them to get their stable, native and functional structure / configuration.

One of the important connections between the amino acids constituting a protein molecule in its tertiary structure is the disulphide bond. This bond is formed exclusively between two cysteines, when present, in a protein molecule. As the number of cysteines in a molecule increases, the possible combinations between them also exponentially increase; but only a few among them are correctly folded, giving the molecule its proper structure and function. So when looking at the above facts it is clear that, when a protein is having multiple cysteines and also expressed as inclusion body in *E. coli,* it is really a Herculean task to get this protein isolated and purified with its structure and function intact and with viable levels of yield even at small scales, without mentioning the industrial scales.

This is applicable to a recombinant protein being a candidate vaccine against Malaria caused by one of the four human malarial parasites, *P. vivax.*

Malaria remains one of the most important global diseases to tackle, affecting more than 150 million people worldwide annually and causing more than 2 million deaths among them, with more than 40% (2 billion people) of the world population at risk. The disease is caused by protozoan parasites belonging to the genus *Plasmodium* with the two species *P. vivax* and *P. falciparum* being the most important ones for humans. Of these two the latter can cause death as it can cross the blood-brain barrier and cause cerebral inflammation. Issues such as (a) the complexity of the parasite's life cycle which traverses two hosts, a vertebrate one in humans and an invertebrate one in female Anopheles mosquitoes, for completion, and (b) presence of a variety of antigenic epitopes displayed by the several life stages of the parasite posing a huge challenge to the host immune system, present a big challenge in the development of an effective vaccine against the parasite. Since all drug-based treatments are not working long- enough consistently, due to the development of resistance by the parasite to most of the drugs used to treat the disease, development of an effective vaccine promises to be the best solution in containing the disease.

The first vaccine developed that has undergone field trials is the SPf66, developed by Manuel Elkin Patarroyo in 1987. It presents a combination of antigens from the sporozoite (using circumsporoziote protein -CSP- repeats) and merozoite stages of parasites. Though phase I trials demonstrated 75% efficacy rate and the vaccine appeared to be well tolerated by subjects and immunogenic, phase lib and III trials were not only less promising with the efficacy falling to between 38.8% and 60.2%, but a trial carried out in Tanzania in 1993 and the most recent (though controversial) study in the Gambia did not show any effect. Thus, despite the relatively long trial periods and the number of studies carried out, it is still not known how the SPf66 vaccine confers immunity, and therefore remains an unlikely solution to malaria.

The CSP was the next vaccine developed that initially appeared promising enough to undergo trials. It is also based on the CSP, but additionally has the recombinant (Asn- Ala-Pro15Asn- Val-Asp-Pro)2-Leu-Arg(R32LR) protein covalently bound to a purified *Pseudomonas aeruginosa* toxin (A9). However, at an early stage a complete lack of protective immunity was demonstrated in those inoculated. The study group used in Kenya had an 82% incidence of parasitaemia whilst the control group only had an 89% incidence. The vaccine intended to cause an increased T-lymphocyte response in those exposed; this was also not observed, thus proving to be ineffective.

The NYVAC-Pf7 multistage vaccine developed incorporating seven *P. falciparum* antigenic genes like CSP and sporozoite surface protein 2 (called PfSSP2) derived from the sporozoite phase, the liver stage antigen 1 (LSA1), three from the erythrocytic stage (merozoite surface protein 1 -MSP1, serine repeat antigen - SERA and apical membrane antigen -AMA-1) and one sexual stage antigen (the 25-kDa Pfs25). This was first investigated using Rhesus monkeys and produced encouraging results. However, trials in humans gave mixed results warranting evidence with regard to efficacy.

In 1995 a field trial involving [NANP] 19-5.1 proved to be very successful. Out of 194 children vaccinated none developed symptomatic malaria in the 12 week follow up period and only 8 failed to have higher levels of antibody present. The vaccine consists of the schizont export protein (5.1) and 19 repeats of the sporozoite surface protein [NANP]. Limitations of the technology exist as it contains only 20% peptide and has low levels of immunogenicity. It also does not contain any immunodominant T-cell epitopes.

RTS,S is the most recently developed recombinant vaccine. It consists of the *P*. *falciparum* CSP protein from the pre-erythrocytic stage. The CSP antigen causes the production of antibodies capable of preventing the invasion of hepatocytes and additionally elicits a cellular response enabling the destruction of infected hepatocytes. The CSP vaccine presented problems in trials due to its poor immunogenicity. The RTS,S attempted to avoid these by fusing the protein with a surface antigen from Hepatitis B, hence creating a more potent and immunogenic vaccine. When tested in trials an emulsion of oil in water and the added adjuvants of monophosphoryl A and QS21 (SBAS2), the vaccine gave protective immunity to 7 out of 8 volunteers challenged with *P. falciparum.*

This clearly indicates that the task of developing a vaccine that is of therapeutic and potentially preventative benefit for malaria is a complex process. No effective vaccine for malaria has so far been developed despite continuous R & D in the area. Hence, there is immense necessity for developing an effective stable vaccine.

The main area of survival of the parasite in the human host is the reticulo-endothelial system which includes organs and tissues such as liver and blood. Most of the growth and maturation of the parasite occurs in the red blood cells of the blood. Two soluble antigens, Duffy Antigen Binding Protein (DABP) and Sialic Acid Binding Protein (SABP), appear in the culture supernatant after infected erythrocytes release merozoites. Immunochemical data indicate that DABP (a ∼135-kDa size protein which specifically binds Duffy blood group determinants) and SABP (a ∼175-kDa protein which binds specifically to glycophorin sialic acid residues on erythrocytes) are the respective ligands for the *P. vivax* and *P. falciparum,* mediating the specific molecular interactions for the invasion of RBCs by malaria parasites. Duffy and sialic acid receptors on erythrocytes possess specificities of binding which are identical either in soluble or membrane bound form.

It was specifically found out that *P. vivax* requires interaction with Duffy blood group antigen for entrance while *P. falciparum* EBA-175 interacts with the sialic acid residues on glycophorin A to mediate erythrocyte invasion. Parasite proteins that bind these RBC receptors to mediate invasion include *P. vivax* Duffy binding protein (PvDBP) and *P. falciparum* sialic acid binding protein (known as EBA-175). The functional erythrocyte binding domains of both proteins were found to lie in a conserved, Cysteine-rich N-terminal regions referred to as *P. vivax* region II (PvRII) and *P. falciparum* region F2 (PfF2).

The *P. vivax* region II (PvRII) was expressed in the BL21 (DE3) strain of *E. coli* [3]. Recombinant PvRII was incorrectly folded upon expression and accumulated as inclusion bodies. These inclusion bodies were solubilized using denaturants, purified under denaturing conditions by metal affinity chromatography, refolded by a rapid dilution method and purified to homogeneity by Ion exchange chromatography to finally obtain milligram quantities of pure, functionally active protein which bound specifically with Duffy positive human erythrocytes.

Rabbits immunized with PvRII thus produced and formulated in Freund's adjuvant, as well as mice immunized with PvRII formulated with the human compatible adjuvants Montanide ISA720 and ASO2A, were shown to develop high-titre binding inhibitory antibodies that block the binding of Duffy positive erythrocytes to COS-7 cells expressing PvRII on their surface. The protective efficacy of recombinant PvRII formulated in Freund's and Montanide ISA720 adjuvants was tested in *Aotus* monkeys. Specific antibody titers were determined by an enzyme-linked immunosorbent assay after each of three doses of 50 micrograms of protein administered by the subcutaneous route. Immunization with PvRII formulated in Freund's adjuvant yielded higher antibody titers than immunization with the Montanide ISA720 formulation and offered partial protection. Although the Montanide ISA720 formulation was immunogenic, it did not provide any protection. Given the immunogenicity and partial protection observed, further research was carried out.

It was observed that, one of the major obstacles for optimal expression of Plasmodium genes in *E. coli* is the difference in codon usage frequency between these two organisms. When the native gene construct was expressed in *E. coli* and purified, few truncated products of PvRII were also co-purified (identified by Western Blotting) and this gave problems in scaling-up the process. A synthetic gene for PvRII with codons optimized for expression in *E. coli* was designed to overcome this existing problem. It has two-fold advantages. (1) Expression of recombinant using synthetic gene gave higher yields when compared with the native gene construct; and (2) the codon optimization significantly reduced the production of truncated PvRII fragments that are observed in case of expression using native gene. A high cell density fermentation to express the protein in high levels was optimized, but the difficulty in getting a good yield of final purified protein remained.

Another major difficulty with production of PvRII is that it has 12 cysteines in its primary structure as shown in Table-1. All these are required to have to be linked-up to get the native structure and functionality. The transcribed product of this synthetic gene is a 336 amino acid, single-chain protein. The cysteine residues are present in positions 25, 38, 45, 54, 108, 185, 223, 235, 240, 244, 313 & 315. Within the chain the critical binding residues for Duffy Antigen Receptor for Chemokines (DARC) lie in the central region of the chain having the cysteine residues 5 to 8.

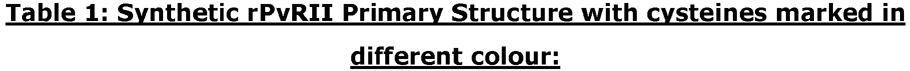

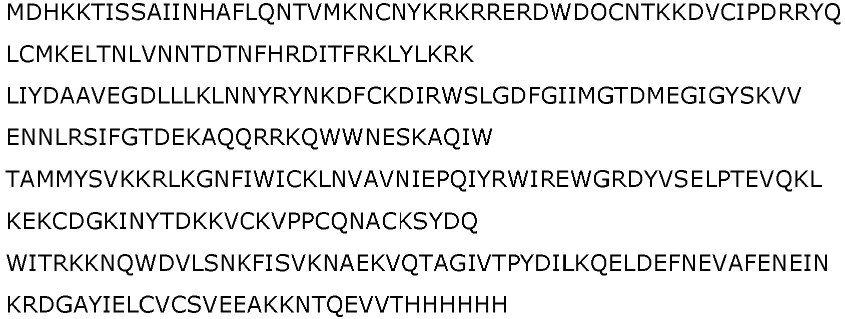

As those who are familiar with the art of renaturation know, presence of 12 cysteines means six disulphide bonds in the chain and the chance of getting the right disulphide linkages in all six cases is very difficult when it is given that the possible combinations of disulphide bonds in this case are many. These difficulties resulted in a process with an unviable yield of less than 5%, though it was enough to test the protein and its functionality. A reference is made to Singh S., K. Pandey, R. Chattopadhayay, S. Yazdani, A. Lynn, A. Bharadwaj, A. Ranjan and C. E. Chitnis. 2001, 'Biochemical, biophysical and functional characterization of bacterially expressed and refolded receptor binding domain of Plasmodium vivax Duffy binding protein', Journal of Biological Chemistry, 276:17111-17116. However, if this had to go to human use higher yields are required. Moreover, the cost of the process also has to be kept low in order to make the product cheaper and affordable for mass use. The synthetic gene construct was taken up for further work to develop a method to have better quality purified, functional PvRII with increase in yield. This is achieved by specially designed method of refolding of protein. It is pertinent to mention here that the modified method disclosed in this invention works well for other proteins with multiple cysteines in their structure and also expressed as inclusion body.

The proteins thus purified are formulated for using as vaccine candidates. During the last two decades, a variety of technologies have been investigated to improve the widely-used adjuvant based on aluminum salts. These salts are unfavorable, since they develop side effects such as inducing local inflammation, which is also the basis for the extended side-effect pattern of this adjuvant. All small-scale animal studies with rPvRII and many of the other information available on recent vaccine trials suggested that though alum, the gold-standard adjuvant, elicited decent response to the antigen(s) when used as the adjuvant, much better responses were observed with other adjuvants. Though one of the important criteria for an adjuvant being that it should not alter the structural / functional characteristics of the active molecule, some of the formulations were shown to be de-stabilizing the proteins due to the interaction with the antigens or by their physical instability, thereby precluding or restricting the usability of the adjuvant. This necessitated new formulation strategies where the benefits of the adjuvant could be used without compromising the stability of the protein or formulation.

The oil-based adjuvants proved to satisfy this requirement. The oil-based adjuvants such as QS21, MPL-A, MF59, ASO2A (SBAS2) and Montanide ISA-720 were used for preparing formulations and evaluated under clinical trials. The newer human adjuvants including MF59, ISCOMS, QS21, AS02 and AS04 have exhibited substantially higher local reactogenicity and systemic toxicity than alum.

As it is apparent from the disclosure herein before, the existing prior art is unable to develop a stable immunogenic protein having multiple cysteines with high purity while maintaining integrity and functionality of native protein due to problems posed by getting appropriate disulphide linkages during refolding of the target protein and co- purification of host protein along with target protein. Other problems associated with the existing prior art are the stability of the protein, low yield of the protein and stability of the formulation of such protein making them unfit for commercially viable and being used effectively as vaccines for long period. The inventor, after prolonged and painstaking R&D, has developed a stable immunogenic protein having multiple cysteines with purity above 98%, stability up to 2 years at low temperature & six months at room temperature, and integrity shown by its functionality during stability studies. Further, the process is industrially feasible with increase in yield from 5% to about 20% and the composition stable over 3 years. The novelty resides in eliminating formation of aggregation, eliminating presence of host proteins and getting appropriate disulphide linkages during refolding of target protein. Additionally, the lyophilization of protein with sucrose before developing formulations adds to the stability of the composition.

S. S. Yazdani et al describe a high-density cultivation method to produce recombinant PvRII in *E. coli* for use in vaccine studies in their publication titled: "A high density fermentation strategy to produce recombinant malarial antigen in E. coli", published in Biotechnology Letters 26: 1891-1895, 2004. Cells were grown in completely defined media and glucose was fed to achieve a specific growth rate of 0.12 h⁻¹ until cells reached 55 g dry wt I⁻¹. Culture was then induced with 1 mM IPTG and cell were further grown for 4 h to reach 85 g dry wt I⁻¹ at 0.1 h⁻¹. Recombinant PvRII was purified from inclusion bodies under denaturing conditions using metal affinity chromatography which yielded 10 mg PvRII g⁻¹ dry wt. After refolding, PvRII was greater than 98% pure, homogeneous and functionally active in that it specifically bound Duffy positive human red blood cells.

In "Preparation and Extraction of Insoluble (Inclusion-Body) Proteins from Escherichia coli", published Current Protocols in Protein Science (2204) 6.3.2-6.3.18, I. Palmer and P.T. Wingfield describe different protocols for the preparation of washed pellets and solubilization of the protein using guanidine^{∗}HCl. The extracted protein, which is unfolded, is either directly folded or further purified by gel filtration in the presence of guanidine^{∗}HCl.

E. Wilharm et al describe in "Generation of Catalytically Active Granzyme K from Escherichia coli Inclusion Bodies and Identification of Efficient Granzyme K Inhibitors in Human Plasma", published in The Journal of Biological Chemistry, Vol. 274, No. 38, pp. 27331-27337, 1999, a refolding and purification of the protein by washing the harvested inclusion bodies twice in 50 mM Tris-HCI, 60 mM EDTA, 1.5 M NaCl, 6% Triton X-100, pH 7.2, followed by two washing steps in 50 mM Tris-HCI, 60 mM EDTA, pH 7.2. The purified inclusion bodies were solubilized in 6 M guanidinium chloride, 100 mM Tris-HCI, 20 mM EDTA, 15 mM GSH, 150 mM GSSG, pH 8.0. Refolding was initiated by diluting the solubilized proteins in 100 volumes of 50 mM Tris-HCI, 0.5 M L-arginine, 20 mM CaCl₂, 1 mM EDTA, 0.1 M NaCl, 0.5 mM L-cysteine, pH 8.5.

R. Rudolph and H. Lilie published a review on "In vitro inclusion body proteins" in FASEB Journal, Vol. 10 in January 1996, where they suggested other low molecular weight thiols than reduced and oxidized glutathione (GSH, GSSG) for folding with concomitant disulfide bond formation, such as cysteine/cystine, cysteamine/cystamine or di-β-hydroxyethyl disulfide/2-mercaptoethanol.

### OBJECTIVE OF THE INVENTION

The object of the present invention is to provide a process for expression, purification and refolding of rPvRII having 12 cysteines useful for prophylaxis of malarial infections in mammals more preferably malarial infections caused by *Plasmodium sp.*

### DESCRIPTION OF THE ACCOMPANYING DRAWINGS:

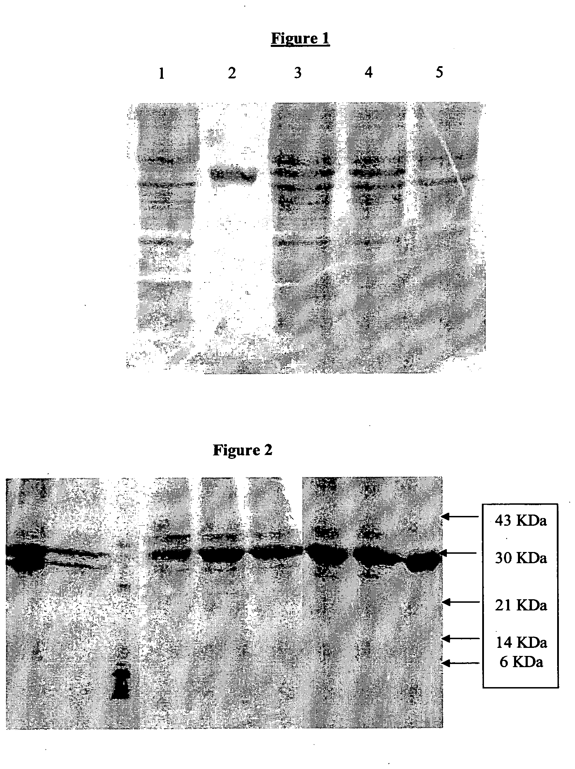

**Figure 1** shows SDS-PAGE profile of induced and un-induced samples of a fermentation batch. The Lanes 1 & 5 show un-induced sample, Lane 2 with Standard PvRII, Lanes 3 & 4 induced sample. The extra band seed in the induced sample Lanes corresponding to the Marker is the rPvRII protein band.
**Figure 2** shows SDS-PAGE profile of fractions from metal affinity chromatography. Lane shows pre-load sample, Lane 2 flow-through, Lane 3 marker, Lane 9 Standard and Lanes 4 to 8 the elutes 1 to 5, respectively.

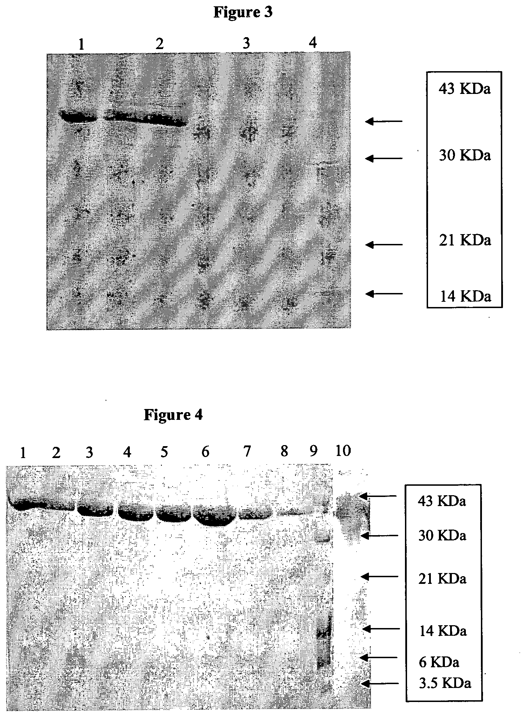

**Figure 3** shows SDS-PAGE profile of post-Refolding, C& DF material. Lane 1 shows Standard, Lane 2 retentate sample, Lane 3 permeate sample and Lane 4 Molecular weight Marker.
**Figure 4** shows SDS-PAGE Profile of "reduced samples' of Ion-Exchange Chromatography run. Lane 1 shows Standard, Lane 9 with Molecular weight Marker, Lane 10 with pre-load sample, Lanes 2 to 8 with elutes 7 to 1 in that order, respectively.

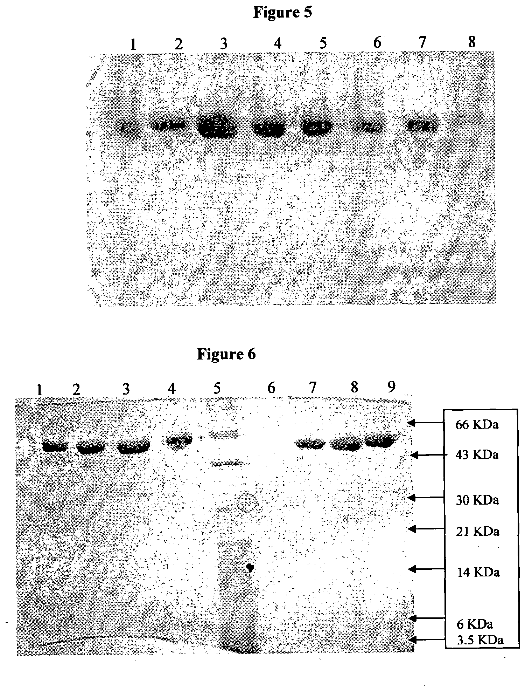

**Figure 5** shows SDS-PAGE Profile of 'non-reduced samples' of Ion-Exchange Chromatography run. Lane 1 shows Standard, Lanes 2 to 8 show elutes 1 to 7 in that order, respectively.
**Figure 6** shows SDS-PAGE Profile of purified rPvRII under reducing and nonreducing conditions. Lane 4 shows Standard (reducing), Lane 7 Standard (nonreducing), Lane 5 Molecular weight Marker, Lanes 1 to 3 bulk antigen (reduced) at 2µg, 4µg and 8µg respectively, Lanes 8 and 9 bulk antigen (non-reduced) at 4µg and 8µg respectively. Lane 6 is blank.

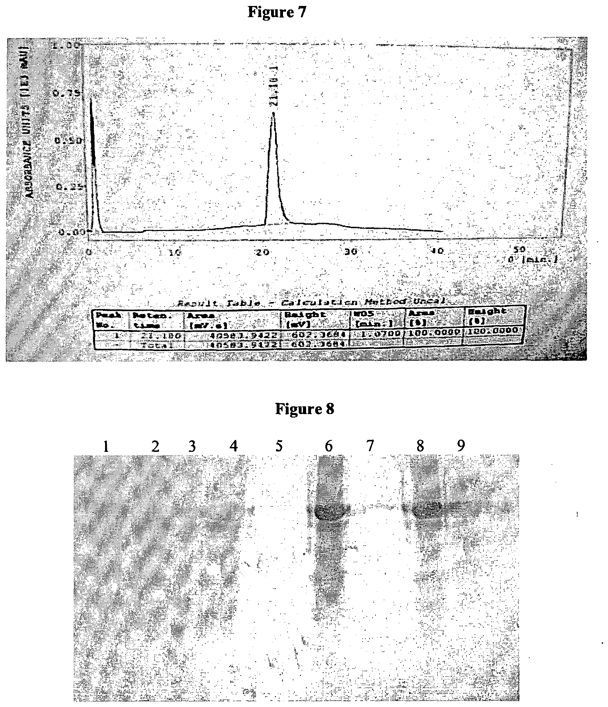

**Figure 7** shows the RP-HPLC profile of purified rPvRII.
**Figure 8** shows the EB A- Western profile of purified rPvRII. Lane 3 shows the Coloured Molecular weight Marker, Lanes 4 & 9 Standard, Lanes 1 & 2 Bulk antigen, Lanes 5 & 7 negative control and Lanes 6 & 8 positive control.
**Figure 9** shows the accelerated and real-time stability profiles of rPvRII bulk antigen. The protein content and purity by RP-HPLC, were plotted as graphs and show that the bulk antigen (protein) is quite stable at the storage conditions tested.
**Figure 10** shows the accelerated and real-time stability profiles of rPvII-alum formulation. The protein content and antigen adsorption estimated by BCA method were plotted as graphs and show that the alum-formulated rPvRII is quite stable at the storage conditions tested.
**Figure 11** shows the accelerated and real-time stability profiles of rPvRII-lyophilized formulation. The protein content and purity by RP-HPLC were plotted as graphs and show that the lyophilized-rPvRII is quite stable at the storage conditions tested.

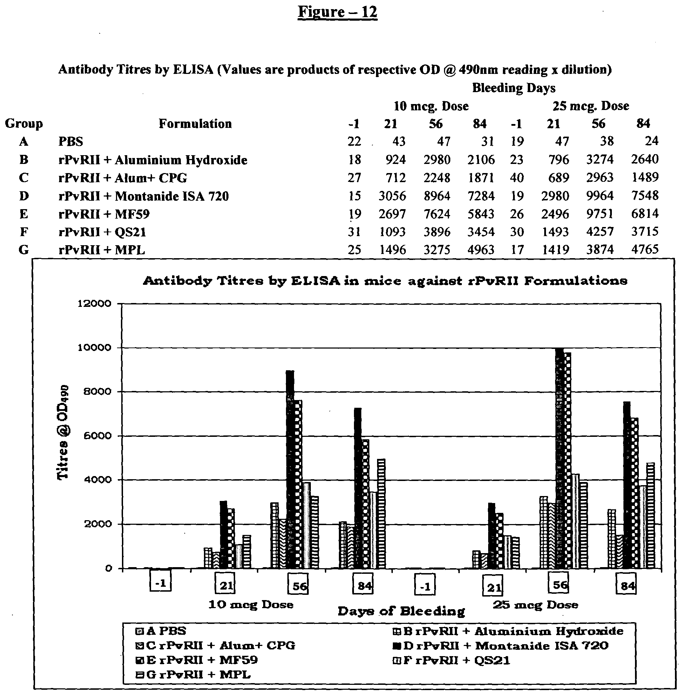

**Figure 12** shows the antibody titres produced by outbred Balb/C mice against different rPvRII+ adjuvant formulations. The antibody titres shown are the end-point titres elicited by the formulated material in sera samples collected from bleedings done at different days pre- and post-inoculation and the OD measured at 490nm.

### STATEMENT OF INVENTION:

Accordingly, provided is a process for the production of a stable immunogenic protein having multiple cysteines molecules wherein the protein is having stability up to two years and purity more than 98%.

The rPvRII protein may be stable for twenty four months at a temperature between 2-8 °C and six months at a room temperature.

The invention provides a method for producing immunogenic protein as disclosed herein before according to claim 1.

According to another embodiment, the harvesting, separating, isolating and solubilizing may be carried out under denaturing conditions.

According to the invention, the refolding is effected employing a buffer containing a detergent - dextrin mixture, a co-solvent and a redox pair.

The detergent used is Triton X-100 at a concentration of 0.1-1.0mM, preferably 0.3-0.7mM; dextrin, preferably cyclodextrin more preferably beta-cyclodextrin at a concentration of 5-20mM and more preferably at 12-18mM; L-Arginine as a co-solvent being added in a concentration of 0.4-0.8M and a redox pair comprising L-cysteine - cystamine dihydrochloride.

According to still other embodiment the refolding buffer further comprising one or more of the amino acids selected from L-cystine, L-cysteine, L-proline and L-lysine.

The re-folded protein may further be purified by column chromatography to be free from chaotropic agent, detergents, monomers and multimers.

### DETAILED DESCRIPTION OF THE INVENTION

The disclosure provides a series of specific steps, with necessary modifications as may be done by those who are competent with the art, to be used in the upstream and downstream processes to obtain the target protein in a pure, active form. The process starts with culturing the host cells containing the synthetic recombinant gene for expressing the target protein, first in seed inoculum stage and using this seed material to inoculate a bioreactor to produce the cells in high density and then inducing the expression of target protein. This expressed protein aggregates as inclusion bodies inside the host cell and these cells are then broken and the inclusion bodies are separated from the lysate, washed and solubilized using a chaotropic agent like guanidine hydrochloride at 6-8M concentration. The solubilized inclusion bodies are clarified by centrifugation and the supernatant is run through metal affinity chromatography and the positive fractions are pooled and concentrated. The concentrated protein is then refolded in the specific combination of buffer components and conditions used in this process; the refolded protein is then concentrated and diafiltered to remove the chaotropic agent and detergents used in the prior steps and run through ion exchange chromatography to purify the monomers from dimers, multimers and other impurities. The pure fractions are then concentrated and sterile filtered before further storage or use.

The final yield of the purified protein in this process ranges from 15% to 25% at different scales of refolding done and well above the levels achieved for such proteins as described in prior art.

### EXAMPLE 1:

### Isolation and Purification of recombinant PvRII expressed as inclusion body in E. coli:

### Fermentation and expression of target protein at high cell concentrations:

The first step in the process sequence is the preparation of inoculum. Seed culture from a vial among the stock of vials preserved at or below -70 °C is inoculated into the fully defined fermentation medium to get high cell density. The inoculum preparation can consist of two or three stages of scale-up before the last-stage inoculum is added to the medium sterilized in situ in the fermentor. Between each transfer samples are taken and checked for cell density, pH and culture purity, the last of which is done by microscopic examination of wet smear or Gram-stained smear of culture, to confirm compliance to specifications; each stage involves transfer of 5% to 10% of inoculum to the succeeding stage, which is grown in a shake-incubator at 150-250 rpm for 8-12 hours at 33 °C-37 °C.

The initial conditions of fermentation are set as follows: Temperature at 37±2 °C, pH at 6.9 ± 0.1, stirring speed at 200-400 rpm and 100% availability of the oxygen through compressed air given at 4 to 8 litres.min⁻¹. The fermentation is monitored continuously and that parameters are controlled, automatically as per pre-set programs or manually as required to be within pre-set limits, which are changed as per requirements during fermentation as known to those conversant with the art. Once the entire carbon source and other nutrients in the medium are exhausted by growing culture, fed-batch cultivation is started by addition of a fed-batch solution containing modified quantities of the nutrients in the basal medium as known to those conversant with fermentation. During this phase the dissolved oxygen (DO) level is maintained between 10% and 50%, preferably between 10% and 30% or 20% and 40%.

During the entire fermentation periodic samples are taken and in-process parameters as required to monitor growth. This fed batch stage is continued until the desired concentration of cells is reached; then the culture is induced with I-2mM of Isopropyl β-D thiogalacto pyranoside (IPTG). During this induction phase the DO level is maintained at a higher level than done during the fed-batch stage as required to improve induction of the target protein. This induction phase extends between 3 and 6 hours, preferably not later than that.

The final OD and PCV after harvest are between 130-160 and 18%-24%, respectively. The final wet biomass obtained ranges from 6 Kgs. to 8 Kg for a 50L batch (110 g. to 160 g^{∗}litre⁻¹) fermentation.

### Harvesting and washing of cells:

For cell harvesting and washing, a Tangential Flow Filtration (TFF) cassette module like PROSTAK of Millipore Corporation, U.S.A, having a porosity of 0.45µm-0.6µm is connected to the fermentor. The flow-rates of permeate and retentate are controlled to get an optimum cross-flow rate. The cells are concentrated to a level of approximately 20% to 40% of their initial volume and then buffer exchanged against washing buffer containing 5-10mM Tris buffer, 2-5mM Disodium salt of Ethylene di-amine tetra- acetic acid (Titriplex), 50-100mM Sodium chloride, pH 7.8±0.2 to the cells following known procedure as practiced by those experienced. The final volume at this stage is to be maintained between 60% and 90% of original harvested cell volumes. The OD of the total cell suspension is then checked and adjusted to be between 80 and 120. During this operation the temperature of the culture is maintained below 20 °C, preferably as required to prevent denaturation of proteins by prolonged exposure to higher temperatures.

### Cell lysis and separation of inclusion bodies:

To the cell suspension under stirring are added a detergent like Triton-X 100 (0.5-1.0%), protease inhibitor like Benzamidine hydrochloride (5-10 mM), Dithiothreitol (DTT, 5-10mM) and Lysozyme (20-30 mg^{∗}litre⁻¹). This cell suspension is stirred for 10 to 20 minutes and lysed by passing it, two to four times, through Dynomill as known to those experienced in the art. Lysis is monitored by checking OD and using microscopy of samples after each pass. After required breakage of the cells, of up to 95% cell lysis, the suspension is centrifuged at 4500 to 5500 x g. for 30 to 60 minutes at 2 to 8 °C and the supernatant is collected separately.

### Washing and solubilization of inclusion bodies:

To one volume of the total pellet about 7-10 volumes (vol. in mL per weight in grams) of IB wash buffer-1 containing 5-10mM Tris buffer, 2-5mM Titriplex, 50-100mM Sodium chloride, 2-4M Urea and 0.5-1.0% Triton X-100, pH 7.3 ± 0.2 is added, stirred for 15 to 30 minutes and centrifuged at 4500 to 5500 x g. for 30-60 minutes at 2-8 °C. The supernatant is collected separately, and the pellets are resuspended in the same buffer as before and the process is repeated once more. To the pellet obtained after the second wash, IB wash buffer-2 containing the constituents as in buffer-1 above minus urea, EDTA and detergent but increased concentration of sodium chloride at 0.5-1 M is added at 3-5 volumes of the total pellet weight, stirred for 15-30 minutes and centrifuged at 4500-5500g. for 30-60 minutes at 2-8 °C. All the washes collected are checked by SDS-PAGE analysis for no or not very significant loss of the target protein in the lysate supernatant and EB washes. **This novel "two buffer** - **three step" wash resulted in removal of most of the host-cell proteins, which have to be within certain limit in bacterial produced proteins to be used in pre-clinical and clinical evaluations, contaminating the target protein in the inclusion bodies.**

The final washed IB pellet is then added with 7-10 volumes of the buffer (5-10mM Tris buffer, 50-100mM Sodium di-hydrogen phosphate, di-hydrate, 50-100mM Sodium chloride, 6-8M Guanidine-HCI, pH 8.0) for solubilizing them and stirred at room temperature (20-25 °C) up to overnight. After this the solubilized material is centrifuged at 4500-5500g. for 60-90 minutes at 2-8 °C. The clear supernatant is collected, checked by SDS-PAGE for the presence of the target protein and then taken for next step.

### Immobilized metal affinity chromatography (IMAC):

The next step is the immobilized metal affinity chromatography using the Streamline chelating matrix and a Streamline column obtained from Amarsham Pharmacia (GE Healthcare), U.S.A. The matrix is charged with 50-100mM Nickel sulphate, washed with distilled water and equilibrated with 3-5 volumes of solubilization buffer. After equilibration, the flow direction is changed to expand the matrix 2-3 times of its original packed height with the sample loaded. The sample is passed for totally 3-5 times through the column, maintaining the expanded state to a constant level. For further washings and elution, the buffer containing 5-10mM Tris, 5-100mM Sodium di-hydrogen phosphate di-hydrate, 6-8M Guanidine-HCI is used with only the change in pH. At the end of the loading, washing of the matrix is done in the expanded state itself using 2-3 (packed bed) volumes of IMAC wash buffer-1 with pH 8.0, followed by washing in the expanded state itself using 5-8 (packed bed) volumes of EMAC wash buffer-2 with pH 6.3. After this washing flow direction is changed and elution is done with 4-5 volumes of the Elution buffer with pH 4.3. Elutes are collected as per requirements to enable fractionation of purified protein using bed-volume as the criteria, in appropriate sterile containers, sampled and analyzed for protein concentration / content and purity by SDS-PAGE. Protein estimation is done by Bradford method and also by checking the sample OD at 280nm and using the molar extinction co-efficient value of 1.815 OD@280nm = 1 mg/mL of the target protein.

IMAC elutes having more than 60% target protein are pooled and the protein concentration and content of the pool is checked and calculated. If required, the elutes are concentrated using 1OkD TFF membrane cassettes to achieve a protein concentration of not less than 3.6 mg/ml. The sample is then is added with 2-1OmM of DTT, mixed and left up to overnight at 2-8 °.C.

### Refolding and diafiltration:

The next step of refolding is done using a chilled (to ∼10 °C) buffer having the following components: 50mM Tris buffer, 1mM Titriplex, 1M Urea, 0.5M Arginine-HCI, 2mM L-cysteine and 0.67mM Cystamine dihydrochloride (the latter two acting as the redox reagents) and 0.57mM of the detergent Triton X-100, pH 7.2 to 7.4. This refolding system was chosen after many trials with different components and compositions; the presence of co-solvent like arginine-HCI and redox pair like Cysteine - Cystamine dihydrochloride were not known to be used along with the detergent - cyclodextrin pair in the total system before. However, when tried in combination only the increases in yields were better. This is probably because of the presence of 12 cysteines and therefore possibly six correct disulphide bonds to be formed in the tertiary structure to confer functionality to the protein. Arginine-HCI, the co-solvent, works like an artificial chaperone and this limits the aggregation, the possibility of whose formation is more as the number of cysteines in the molecule increase. The concentration of arginine-HCI tried in the system carried from 0.2M to 1.0M and there was significant increase in the refolding efficiency up to 0.5M concentrations and after that up to 0.8M levels there was not much increase and continuing on up to IM levels resulted in slight drop in the refolding efficiency. The novel redox pair of cysteine - cystamine dihydrochloride helps in formation and reshuffling of the disulphide bonds. The use of other redox pairs like GSSG:GSST (oxidized and reduced glutathione), and other detergents tried like CTAB (cetyl-trimethyl ammonium bromide) in the buffer system did not give similar yields in parallel experiments done to optimize the conditions of refolding (data not shown). The pH used was about 1.0 to 2.0 pH points down from the pi of the target protein (here the pi of PvRII is ∼8.9). The same refolding buffer-complex was used to check refolding at different temperatures, viz. 4 °C, 10 °C and 25 °C to check for efficiency. The one done at 10 °C worked better than the other two done at lower and higher temperatures. The concentration of protein added into the system for refolding was also checked at various levels, from 60 mg^{∗}litre⁻¹ to 240 mg^{∗}litre⁻¹ and level mentioned above was chosen based on the better results obtained in the experiments. Two other methods with change of components as follows but keeping the protein concentration and temperature same as above were tried.

The DTT-treated protein at a concentration of at least 3.6 mg/mL is added to the buffer under stirring in a vessel, for a final concentration of 120 mg^{∗}litre⁻¹. After this mixture is stirred for 10-20 minutes to ensure complete mixing of the protein in the buffer, 16mM beta-cyclodextrin solution is added under stirring to it for a final concentration of 4.8mM. The air inside the vessel is flushed-out using Nitrogen gas purging for a few minutes to prevent any unwanted effect on the redox potential. This reaction with mild stirring continues for about 20-28 hours at ∼10 °C.

After this reaction the refolding mixture is subjected to a concentration and diafiltration step using 10-kDa TFF membrane cassettes and to about 15%-25% of the initial volume and then brought into 50mM Phosphate buffer containing IM Urea, pH 7.6 to 8.0. The final volume of the concentrated and diafiltered refolded protein would be about 20%-30% of initial volume. This material is filtered through a 20-50µm membrane pre-filter to remove any aggregates, sampled and then taken for the next step.

### Ion exchange chromatography / Diafiltration / Sterile Filtration:

The next step is the anion exchange chromatography, using Toyopearl-SP 650C as the matrix and 50mM Phosphate buffer. The refolded, diafiltered and pre-filtered sample is loaded onto the matrix. Washing and elution are done using step gradient of NaCl concentration, from 50mM to 500mM, in phosphate buffer. Volumes of all the fractions collected range from 0.5 to 2 bed volumes according to the requirement. These fractions including the flow-through and washes are analyzed for protein concentration using OD@280nm (1.834 OD@280 = mg/mL of native target protein) and/or BCA assay and purity by SDS-PAGE. Fractions having mostly only the target protein are pooled and then concentrated and diafiltered into 10mM Phosphate buffered saline using 10-kDa TFF cassette. The final concentrated protein is checked as above for concentration and purity. The final protein bulk is then sterile-filtered using 0.22 µm modified PVDF membranes (capsule filter) and stored at 2-8 °C.

The final product yield from the levels expressed was about 17% to 25%, much higher than the 5% mentioned for this protein in earlier process shown in the prior art and the final purity of the target protein is more than 98%.

**Table - 2: Results of Yields for different Refolding Methods followed**

| Refolding Method Description as in | Protein Cone. Used for RF | Ion-Ex. Yield (%) | Final Yield (%) |
|---|---|---|---|
| Example 1 | 4.80 mg/ml | 24.5 % | 22.5 % |

### EXAMPLE 2: Characterization of the final bulk protein:

The final proteins obtained using all the refolding methods above were analyzed for protein content by BCA assay and/or OD@280nm and/or RP-HPLC. The purity was checked by SDS-PAGE and RP-HPLC assays. Each bulk was also checked for extraneous contaminants like host-cell proteins and nucleic acids, endotoxins, residual amount of nickel (by fluorimetric analysis) and presence of any free cysteine by Ellman Test.

The most important functional assay done to check the nativity and integrity of the protein was the erythrocyte binding assay. In this assay, about 10 meg. of the purified protein was bound to washed human erythrocytes for about an hour at room temperature. The erythrocytes were then washed to remove any unbound material and then the bound protein was eluted using 1.5M NaCl. This eluted fraction was run on SDS-PAGE gel, transferred to a nitrocellulose or modified PVDF membrane and then treated with primary and secondary antibodies (Western blotting); finally after development of the membrane using the appropriate substrate the transferred protein bands could be seen. Presence of protein band at the level of the target protein in eluted samples indicated proper functionality. Control erythrocytes treated with chymotrypsin to remove the Duffy antigen from their surface and so should not bind any PvRII (Duffy Binding Protein) present in sample and so should not show any band in Western blotting. The purified protein consistently passed all the characterization tests to confirm is purity, strength and activity.

### Stability studies:

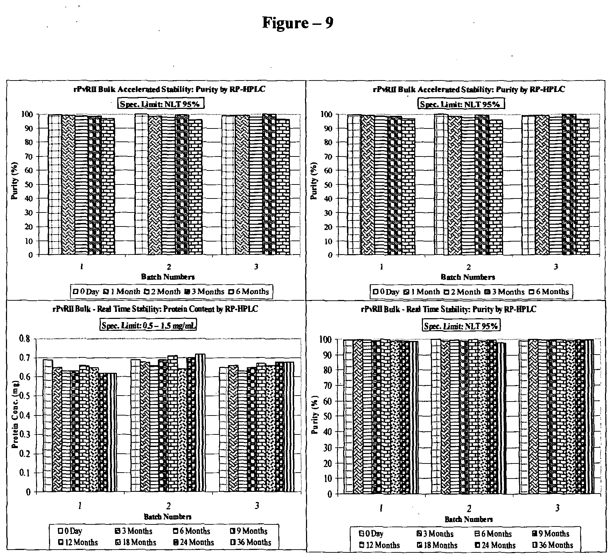

Three batches of this protein were subjected to stability studies in glass containers at the final concentration (between 0.5 and 1.5 mg/mL) obtained at 2-8 °C for 24 months and at 25±1 °C (accelerated study) for 6 months. The parameters analyzed to check stability were Physical description, pH, Protein content & Purity by Reverse Phase HPLC (RP-HPLC), Protein Profile by SDS-PAGE, EBA, Sterility and Bacterial Endotoxin Content by LAL assay. In both conditions of storage, the protein was found to be stable, maintaining its sterility, purity, integrity and functionality. Some profiles of the assay values of the rPvRII bulk antigen are given in Figure 9.

EXAMPLE 3:

### Formulations of the protein:

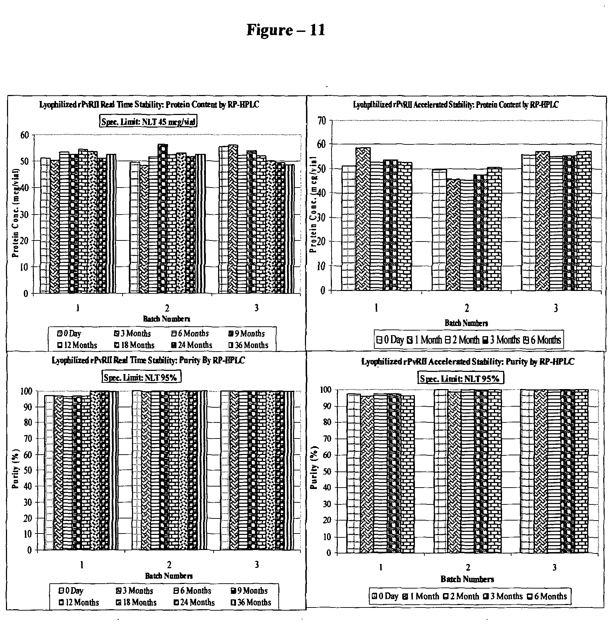

Two formulations of purified protein [one with alum (aluminium hydroxide), the gold- standard adjuvant, and the other a lyophilized one with sugar] were made. For the lyophilized formulation, the composition and cycle were optimized for the protein. The required concentration of protein was lyophilized with sucrose as the stabilizer. The freezing was done to below -50 °C for up to 6-8 hours; primary drying was done in stages between the temperatures of -55 °C up to +23 °C for a time of 31-37 hours; the secondary drying was done at a temperature between +23 °C and +25 °C for a time of up to 6-8 hours. The composition was stable as shown by the study done up to 3 years of storage at 2-8 °C (refer Figure 11). This was done to prevent stability issues that have arisen due to pre-mixing of antigens with the new-age oil- based adjuvants mentioned in literature, and to harness the immune-enhancing power of the latter in eliciting memory responses and high antibody titres.

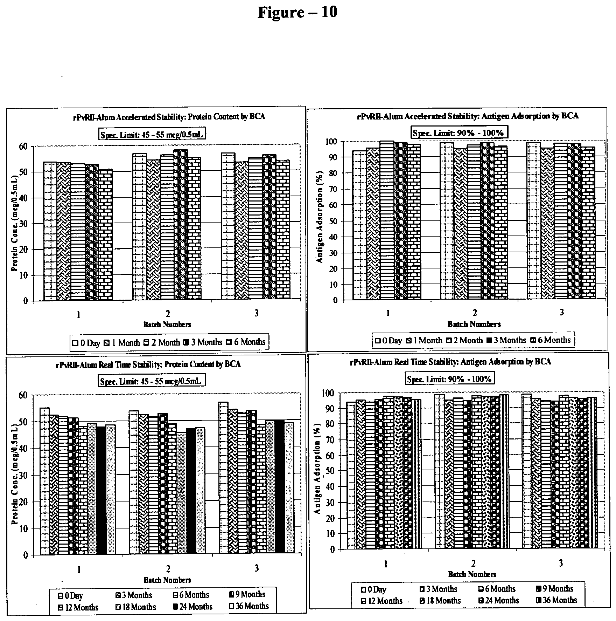

Both the formulations were checked for storage stability at 2-8 °C for 36 months and at 25±1 °C (accelerated study) for 6 months. The parameters analyzed to check stability of alum formulation were Physical description, pH, Protein content by BCA, Protein Profile by SDS-PAGE, Western Blotting, Sterility and Pyrogen Testing. The parameters analyzed to check stability of lyophilized composition were Physical description, pH, Protein content & Purity by Reverse Phase HPLC (RP-HPLC), Protein Profile by SDS-PAGE, EBA, Moisture content, Sterility and Bacterial Endotoxin Content by LAL assay. In both conditions of storage, both formulations of the protein were found to be stable, maintaining the sterility, purity, integrity and functionality. Some profiles of the assay values of the formulated material are given in Figures 10 & 11.

Both the above compositions were then tested in various combinations with other adjuvants for evaluating immunogenicity.

The alum formulation was kept as the base. A second formulation was alum with the non-CpG molecule (IMT-504). For the other formulations, the lyophilized composition was used with various adjuvants as follows: Montanide ISA-720 (a highly refined emulsifier from the mannide monooleate family in a natural metabolizable oil solution from Seppic, France), MF-59 (a type of squalene-oil-water emulsion), MPL (mono- phosphoryl lipid A) and QS-21 (a water-soluble triterpene glucoside compound of saponin family extracted from *Quillaja saponaria*).

The alum-based formulations had the protein at the required dosage level in phosphate buffered saline, pH 7.2±0.2 with alum at a concentration not more than 1.25mg/dose as AlOH₃, all components mixed together by stirring. The alum-IMT 504 formulation had the latter added to the alum formulation at 50 to 100 microgram concentration per dose.

For other formulations, the lyophilized composition was mixed with the respective oil- based adjuvant so as to form a stable emulsion; this was obtained using a double- syringe system with inter-connection between the syringes. The protein and the adjuvant were mixed in required ratios and passed under pressure between the syringes to form an emulsion and *this formulation was used within a maximum of 12 hours.*

For these studies, formulations of the bulk / lyophilized rPvRII as appropriate for the adjuvant used were made to give two different final dosage concentrations of 10 µg and 25 µg. Inbred Balb/C mice were used for the study, chosen based on earlier experimental data. For each formulation one group of 5 mice were used, with one control group which was administered with PBS diluent. Two doses of the dosage concentrations in 0.5mL volumes, on days'0' and '28,' were give intramuscularly. Animals were bled on days -1, 21, 56 and 84. Sera were collected from each sample; sera from each group and each bleed were separately pooled and stored at -20 °C until analysis.

ELISA method was used to estimate the antibody titres in the serum samples, using plates coated with 2µg of purified rPvRII antigen. After blocking the coated-plates, several dilutions of the sera starting from 'neat sample (0)' through 1000, 2000, 4000, 8000 and 12000 were added to the assigned wells and incubated for 90-120 minutes at 37 °C under mild shaking. After washing the wells post-incubation using PBS/Tween-20, Goat anti-mouse HRPO-conjugated secondary antibody was added to the wells and again incubated for 60-90 minutes at 37°C under mild shaking. After washing the wells post-incubation using PBS/Tween-20, the substrate solution containing Diamino-benzidine (DAB) was added to each well and incubated under mild shaking at 25-30 °C for 5 minutes. 2N sulphuric acid was then added to each well to stop colour development and the plates were read at 490nm using an ELISA reader. The end-point titres were then calculated using the readings obtained. The results were then plotted using MS-Excel programme to check and compare the immunogenic responses.

Though most of the oil-based adjuvants, including the Montanide ISA-720 mentioned above, have been tried with the many vaccine-candidate molecules, the novelty is in the formulation strategy of using a lyophilized preparation with the adjuvants to enable mixing the adjuvants with the antigen and administering within a short time. This precludes any possible stability issues and also helps in eliciting good immunogenic responses in animal studies as shown by the data given in Figure - 12.

All the formulations were shown to elicit high antibody titres, with the order of response from high to low being as follows: Montanide ISA720 > MF59 > MPL > QS21 > Alum > Alum+CPG. These responses were consistent with earlier available results of rPvRII produced involving different refolding process.

The stability study results of the bulk antigen / formulations and the efficacy results of the formulations reveal that the process developed for the expression and purification of the protein had worked very well and had given a stable and quality protein; and the formulation strategy used had clearly overcome the stability issues related to the adjuvants used and resulted in very good immune responses to the antigen.

## Claims

1. Process for expression, purification and refolding of recombinant *Plasmodium vivax* Duffy binding protein region II (rPvRII) having multiple cysteines useful for the prophylaxis of malarial infection in mammals, the process comprising the following steps:
a) culturing the host *E. coli* cells containing a desired recombinant gene construct comprising a codon optimized gene sequence of rPvRII,
wherein rPvRII has the structure: 
MDHKKTISSAIINHAFLQNTVMKNCNYKRKRRERDWDCNTKKDVCIPDRRYQ
LCMKELTNLVNNTDTNFHRDITFRKLYLKRK
LIYDAAVEGDLLLKLNNYRYNKDFCKDIRWSLGDFGDIIMGTDMEGIGYSKVV
ENNLRSIFGTDEKAQQRRKQWWNESKAQIW
TAMMYSVKKRLKGNFIWICKLNVAVNFFIPQIYRWIREWGRDYVSELPTEVQKL
KEKCDGKINYTDKKVCKVPPCQNACKSYDQ
WITRKKNQWDVLSNKFISVKNAEKVQTAGIVTPYDILKQELDEFNEVAFENEIN
KRDGAYIELCVCSVEEAKKNTQEWTHHHHHH
 to produce cells in high density
b) inducing expression of rPvRII in *E. coli* as inclusion bodies,
c) harvesting the cells and isolating the said inclusion bodies,
d) separating rPvRII from inclusion bodies by repeated sequential washing with washing with a buffer, the buffer used being a low salt buffer consisting of 5-50 mM Tris, 2-25 mM EDTA, 50-100 mM sodium chloride, 1-6 M urea, 0.1-1% Triton X-100, pH 6.5-7.5; followed by washing with a high salt buffer containing 5-50 mM Tris, pH 6.5-7.5 and 0.5-1 M sodium chloride;
and solubilizing with chaotropic agents comprising guanidine hydrochloride at 6-8 M concentration,
e) purifying the protein by subjecting to metal-chelate affinity chromatography,
f) re-folding of the purified rPvRII obtained in step e) by employing a buffer containing a detergent - dextrin mixture, a co-solvent and a redox pair, wherein the detergent used is Triton X-100 at a concentration of 0.1-1.0 mM, a dextrin at a concentration of 5-20 mM, L-Arginine as a co-solvent being added in a concentration of 0.4-0.8 M and a redox pair comprising L-cysteine - cystamine dihydrochloride; followed by
g) further purifying the desired protein by removing impurities by subjecting to chromatography.

2. A process as claimed in claim 1 wherein the harvesting, separating, isolating and solubilizing is carried out under denaturing conditions.

3. A method as claimed in 1, wherein the refolding buffer further comprises one or more of the amino acids selected from L-cystine, L-cysteine, L-proline and L-lysine.

## Patentansprüche

1. Verfahren zur Expression, Reinigung und Umfaltung von rekombinantem *Plasmodium-vivax*-Duffy-Bindungsprotein-Bereich II (rPvRII) mit mehreren Cysteinresten, der für die Prophylaxe einer Malariainfektion bei Säugern geeignet ist, wobei das Verfahren die folgenden Schritte umfasst:
a) Kultivieren der *E.-coli*-Wirtszellen, die ein gewünschtes rekombinantes Genkonstrukt enthalten, das eine codonoptimierte Gensequenz von rPvRII umfasst, wobei rPvRII die folgende Struktur aufweist: 
MDHKKTISSAIINHAFLQNTVMKNCNYKRKRRERDWDCNTKKDVCIPDRRYQ
LCMKELTNLVNNTDTNFHRDITFRKLYLKRK
LIYDAAVEGDLLLKLNNYRYNKDFCKDIRWSLGDFGDIIMGTDMEGIGYSKVV
ENNLRSIFGTDEKAQQRRKQWWNESKAQIW
TAMMYSVKKRLKGNFIWICKLNVAVNFFIPQIYRWIREWGRDYVSELPTEVQKL
KEKCDGKINYTDKKVCKVPPCQNACKSYDQ
WITRKKNQWDVLSNKFISVKNAEKVQTAGIVTPYDILKQELDEFNEVAFENEIN
KRDGAYIELCVCSVEEAKKNTQEWTHHHHHH
 wobei Zellen in hoher Dichte produziert werden;
b) Induzieren der Expression von rPvRII in *E. coli* als Inklusionskörper;
c) Ernten der Zellen und Isolieren der Inklusionskörper;
d) Abtrennen des rPvRII aus den Inklusionskörpern durch wiederholtes sequentielles Waschen, und zwar Waschen mit einem Puffer, wobei der verwendete Puffer ein Niedersalzpuffer ist, der aus 5-50 mM Tris, 2-25 mM EDTA, 50-100 mM Natriumchlorid, 1-6 M Harnstoff, 0,1-1% Triton X-100, pH 6,5-7,5, besteht; und anschließendes Waschen mit einem Hochsalzpuffer, der 5-50 mM Tris, pH 6,5-7,5, und 0,5-1 M Natriumchlorid enthält;
und Löslichmachen mit chaotropen Mitteln, die Guanidin-Hydrochlorid in einer Konzentration von 6-8 M umfassen;
e) Reinigen des Proteins durch Metallchelat-Affinitätschromatographie;
f) Umfalten des in Schritt e) erhaltenen gereinigten rPvRII durch Einsatz eines Puffers, der ein Tensid-Dextrin-Gemisch, ein Cosolvens und ein Redoxpaar enthält, wobei es sich bei dem verwendeten Tensid um Triton X-100 in einer Konzentration von 0,1-1,0 mM handelt und ein Dextrin in einer Konzentration von 5-20 mM vorliegt, wobei L-Arginin als Cosolvens in einer Konzentration von 0,4-0,8 M hinzugefügt wird und eines Redoxpaars, das L-Cystein/Cystamin-Dihydrochlorid umfasst; und anschließend
g) weiteres Reinigen des gewünschten Proteins durch Entfernen von Verunreinigungen durch Chromatographie.

2. Verfahren gemäß Anspruch 1, wobei das Ernten, Abtrennen, Isolieren und Löslichmachen unter Denaturierungsbedingungen durchgeführt werden.

3. Verfahren gemäß Anspruch 1, wobei der Umfaltungspuffer weiterhin eine oder mehrere der Aminosäuren umfasst, die aus L-Cystin, L-Cystein, L-Prolin und L-Lysin ausgewählt sind.

## Revendications

1. Procédé pour exprimer, purifier et replier la région II de la protéine de liaison à l'antigène Duffy de *Plasmodium vivax,* recombinante (rPvRII), présentant des cystéines multiples, appropriée pour la prophylaxie des infections de malaria chez les mammifères, le procédé comprenant les étapes suivantes consistant à :
a) cultiver les cellules hôtes d'*E*. *coli* contenant une construction génétique recombinante désirée qui comprend une séquence génétique à codons optimisés de rPvRII,
où rPvRII présente la structure suivante : 
MDHKKTISSAIINHAFLQNTVMKNCNYKRKRRERDWDCNTKKDVCIPDRRYQ
LCMKELTNLVNNTDTNFHRDITFRKLYLKRK
LIYDAAVEGDLLLKLNNYRYNKDFCKDIRWSLGDFGDIIMGTDMEGIGYSKVV
ENNLRSIFGTDEKAQQRRKQWWNESKAQIW
TAMMYSVKKRLKGNFIWICKLNVAVNFFIPQIYRWIREWGRDYVSELPTEVQKL
KEKCDGKINYTDKKVCKVPPCQNACKSYDQ
WITRKKNQWDVLSNKFISVKNAEKVQTAGIVTPYDILKQELDEFNEVAFENEIN
KRDGAYIELCVCSVEEAKKNTQEWTHHHHHH
 pour produire des cellules en haute densité,
b) induire l'expression de rPvRII dans *E. coli* en tant que corps d'inclusion,
c) récolter les cellules et isoler lesdits corps d'inclusion,
d) séparer le rPvRII desdits corps d'inclusion par lavage séquentiel répété, par lavage avec un tampon, le tampon utilisé étant un tampon de faible force ionique consistant en 5-50 mM Tris, 2-25 mM EDTA, 50-100 mM chlorure de sodium, 1-6 M urée, 0,1-1 % Triton X-100, pH 6,5-7,5, et ensuite lavage avec un tampon de haute force ionique contenant 5-50 mM Tris, pH 6,5-7,5, et 0,5-1 M chlorure de sodium, et
solubiliser avec des agents chaotropes comprenant du hydrochlorure de guanidine à une concentration de 6-8 M,
e) purifier la protéine en la soumettant à une chromatographie d'affinité aux chélates métalliques,
f) replier du rPvRII purifié obtenu dans l'étape e) en utilisant un tampon contenant un mélange de tensioactif/dextrine, un cosolvant et une paire redox, dans lequel le tensioactif utilisé est le Triton X-100 à une concentration de 0,1-1,0 mM, une dextrine à une concentration de 5-20 mM, L-arginine comme cosolvant étant ajoutée à une concentration de 0,4-0,8 M, et une paire redox comprenant L-cystéine/dihydrochlorure de cystamine; et ensuite
g) purifier encore la protéine désirée en éliminant des impuretés en la soumettant à une chromatographie.

2. Procédé selon la revendication 1, dans lequel lesdites étapes consistant à récolter, séparer, isoler et solubiliser sont effectuées sous des conditions dénaturantes.

3. Procédé selon la revendication 1, dans lequel le tampon de repliage comprend en outre un ou plusieurs des acides aminés choisis parmi L-cystine, L-cystéine, L-proline et L-lysine.
